# EUROPEAN PATENT SPECIFICATION

(11) **EP 1 553 869 B1**
(45) Date of publication and mention of the grant of the patent: **24.06.2015**
(21) Application number: 03753807.1
(22) Date of filing: 16.10.2003
(51) Int. Cl.: A61B 5/11, A61B 5/0432

(54) **CARDIAC MONITORING APPARATUS AND METHOD**
HERZÜBERWACHUNGSVORRICHTUNG UND VERFAHREN
APPAREIL ET PROCEDE DE SURVEILLANCE CARDIAQUE

(30) Priority: 18.10.2002 GB 0224299
(43) Date of publication of application: 20.07.2005
(73) Proprietor: CamNtech Limited, Papworth Everard Cambridge CB23 3UY (GB)
(72) Inventor: UNGLESS, Gary Steven, Papworth Evverard, Cambridgeshire CB38U (GB); OAKLEY, Nigel, Robert, Papworth Evverard, Cambridgeshire CB38U (GB)
(74) Representative: Goodman, Simon John Nye
(86) International application number: PCT/GB2003/004487
(87) International publication number: WO 2004/034896

(56) References cited:
- EP-A- 0 513 548
- EP-A- 0 925 756
- GB-A- 2 207 579
- JP-U- 62 202 804
- US-A- 6 117 077
- US-B1- 6 441 747
- PATENT ABSTRACTS OF JAPAN vol. 2000, no. 24, 11 May 2001 (2001-05-11) & JP 2001 198096 A (TERUMO CORP), 24 July 2001 (2001-07-24)
- MAKIKAWA M; IMAI K; SHINDOI T; TANOOKA K; IIZUMI H; MITANI H: "Microprocessor-based memory device for ambulatory heart rate and physical activity recording" METHODS OF INFORMATION IN MEDICINE, ISSN 0026-1270, vol. 33, no. 1, March 1994 (1994-03), pages 94-96, XP008027621 German
- TONG, DAVID A: "Investigation into Electrocardiogram Motion Artifact Reduction, 10-9191" , [Online] 1 April 2000 (2000-04-01), XP002270312 IR&D Southwest Research Institute, San Antonio, TX, USA Retrieved from the Internet: <URL:http://www.swri.edu/3pubs/IRD2001/10- 9191.htm> [retrieved on 2004-02-12]
- RAYA M A D ; SISON L G : " Adaptive noise cancelling of motion artifact in stress ECG signals using accelerometer " CONFERENCE PROCEEDINGS. 24TH ANNUAL INTERNATIONAL CONFERENCE OF THE ENGINEERING IN MEDICINE AND BIOLOGY SOCIETY. ANNUAL FALL MEETING OF THE BIOMEDICAL ENGINEERING SOCIETY, HOUSTON, TX, USA, 23-26 OCT. 2002, ISBN 0-7803-7612-9, vol. 2, 2002, pages 1756-1757, XP002270313 Pisacataway, NJ, USA, IEEE, USA

## Description

This invention relates to a cardiac monitoring apparatus and method for monitoring a user's heart rate, or other parameters derived from heart-beat sensing.

Heart rate is a physiological parameter that is measured in a wide variety of situations, for example to determine the health status and fitness of a person or animal. It can be used, for example, to give a measure of energy expenditure of an individual and a number of devices exist for doing this by converting heart rate to calories used. Many conventional systems comprise a belt worn around a user's chest and carrying a heart-beat sensor and a radio transmitter for transmitting measured data to a wrist-worn display unit.

Such conventional systems are generally uncomfortable or impractical to wear for extended periods and also suffer a significant problem in that correlating heart rate with calories used may only be effective for exercise rates achieving significant heart rate increases. Smaller increases in heart rate can be due to, for example, stress rather than physical exertion and may therefore be misinterpreted by conventional heart-rate monitoring system.

Documents GB 2207579 A, US 6117077 A1 and US 5716380 A1 disclose some common ECG monitors.

The invention provides an apparatus and a method for monitoring a user's heart as defined in the appended independent claims. Preferred or advantageous features of the invention are set out in dependent subclaims.

The invention may thus advantageously provide a monitor for monitoring a user's heart, comprising a cardiac sensor and a support means for mounting the monitor only on a single adhesive pad, which is preferably an electrocardiogram (ECG) electrode. The monitor is then advantageously both physically supported by the single electrode and electrically connected to it for receiving signals for monitoring the user's heart. A lead extends from the monitor to receive signals from a second ECG electrode, spaced from the first, to enable cardiac monitoring. Further leads coupling the monitor to further ECG electrodes may advantageously allow monitoring of more than one ECG channel. The leads may either extend from a monitor housing or plug into sockets in the monitor housing.

The monitor may comprise a memory for recording data from the cardiac sensor over a period of time, which may then be downloaded for analysis. If more than one ECG channel is to be monitored, it preferably contains sufficient memory to store data from multiple channels for an extended period, such as 24 hours or more. Since the monitor is advantageously of light weight and small size, its housing preferably being of smaller lateral dimension than an ECG pad, it may advantageously be comfortable to wear continuously for periods of as long as days or more.

In a preferred embodiment, the apparatus of the invention consists of a small, lightweight monitor that may measure not only heart rate but also inter-beat interval and/or other cardiac parameters.

The following description of the invention refers mainly to a preferred embodiment in which the monitor further comprises an accelerometer for actigraph measurement. It should be noted, however, that the skilled person will be readily able to identify and assess those features and advantages of this embodiment which similarly apply to the cardiac monitor mounted on a single ECG pad described above.

In the preferred embodiment, the invention may advantageously provide a monitor comprising a heart-beat sensor and an accelerometer which can be secured in position on a single adhesive pad for sensing a user's heart-beat and movement, or activity. The monitor further comprises a processor for receiving signals from the heart-beat sensor and the accelerometer and for generating heart-rate or other cardiac data, and movement or activity data. The monitor preferably comprises a memory in which the data can be stored.

The monitoring of both a user's heart rate and movement addresses the problem outlined above, that heart rate increases are not necessarily correlated to physical exertion. Thus, a record of the user's movement can be correlated with heart rate measurements to improve evaluation of the user's energy expenditure. The range of uses for such an apparatus or method in the medical field is widespread. For instance, it can be used in cardiology, sleep medicine, diabetes, obesity, eating disorders, psychiatric disorders etc. It can also be used in monitoring the fitness levels of individuals and as a means for assessing their energy expenditure. This may be done for a variety of reasons, such as weight loss, rehabilitation, encouragement to exercise etc.

The monitor is preferably couplable to a conventional adhesive electrocardiogram (ECG) electrode or pad attachable to the user's chest. Two such ECG electrodes are preferably used, as in conventional ECG measurement. The monitor may then clip directly to one of the ECG electrodes, achieving both electrical connection and mechanical support. An electrical lead may then couple the monitor to the other ECG electrode.

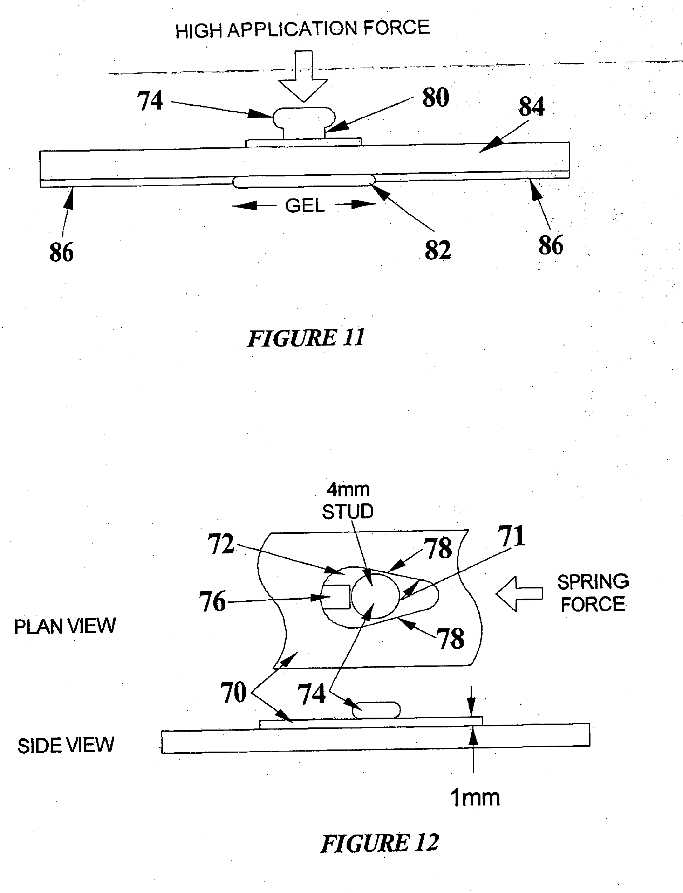

Many different types of ECG electrode pads are available for many different applications, for example to cater for different patient skin types, shapes, and sensitivity, or for long term or short term applications. Thus, some types of electrode have adhesive allowing for easy removal after short term use and others have stronger adhesive allowing for long term skin adhesion. As illustrated in Figure 11 some electrode types comprise an electrode gel 82 beneath a central portion of the pad 84 to improve electrical connection to the skin, surrounded by an adhesive portion 86 of the pad which secures the pad to the skin and retains the gel in position. ECG pads are typically fitted with a standard 4mm stud 74, couplable to an ECG lead. A monitor that mounts directly on to an ECG electrode should preferably be compatible with all these types of pads.

Preferably, a zero insertion force clip is used to connect the monitor embodying the invention to a conventional ECG electrode. If an ECG electrode comprises an electrode gel as described above, and a large force is applied to connect the monitor to such a pad, then this gel may be forced out past the adhesive surrounding it. Advantageously, a zero insertion force clip, for example a slider clip, may prevent the gel being forced out. A high application force for attaching a monitor to a clip may also cause the ECG electrode to deform thereby making attachment of the monitor difficult or even damaging to the electrode. This problem may be exacerbated if the user, or patient, has normal or large amounts of body fat. A zero insertion force clip may, advantageously, make the monitor easier to mount and prevent distortion of the ECG electrode during mounting of the monitor.

Preferably, the unit is securely fixed to the ECG electrode pad so that it will not rotate on the pad during normal use. Any rotation of the monitor on the pad may cause a loss of resolution of the movement (preferably vertical movement) detected by the accelerometer. Advantageously, a clip with a high clamping force may reduce rotation on the pad during normal use. A high clamping force may also, advantageously, reduce contact resistance problems. These factors may favour a high clamping force and so may further exacerbate the problems arising if a zero-insertion-force clip is not used, because otherwise a high clamping force typically requires a high insertion force. In a preferred embodiment, therefore, the monitor is attached to the electrode pad by means of a zero insertion force slider clip, clamping force being provided by a spring contact and the spring being manually retracted as the monitor is attached.

Being small and of light weight, the monitor is advantageously unobtrusive and can be worn for long periods by people of all ages and health or fitness status.

Data from the heart-beat sensor and the accelerometer are advantageously stored in a memory within the monitor, which negates the need for radio or other transmission of data from the monitor. Data may then be downloaded from the monitor by interfacing it to, for example, a computer such as a PC. The monitor interfaces to the PC through the same contacts as used for couplinq to the ECG electrodes. Particularly advantageously, the same contacts may also be used for charging a battery within the monitor.

By analysing data downloaded to a PC, it may advantageously be possible to establish whether small but significant changes in heart rate (usually increases in heart rate) are due to physical exertion or not, and therefore whether heart rate increases may be due to, for example, stress. This may improve estimation of energy expenditure derived from physical activity and its consequences in terms of heart rate and performance.

Alternatively, by identifying changes in heart rate which are not associated with physical activity, conditions such as stress may be identified and/or monitored.

In another embodiment, the monitor may interface with an external device such as a wrist-worn actigraphy device, by means of radio transmission. In this embodiment, the ECG input lead may be used as an antenna for RF output. Heart rate and activity data may then be transmitted to a wrist-worn actigraphy device. Advantageously, for example this wrist-worn actigraphy device may sense reduced upper-body activity whilst the user is seated and engaged in activities such as typing or knitting, which might otherwise cause a wrist-worn actigraph, on detecting significant movement, to estimate an inaccurately high level of energy expenditure. The device may then combine all the data to further enhance the measurement of physical activity and stress. More accurate measurements may be made using both chest-worn and wrist-worn actigraphs in this way, but in alternative embodiments other systems may be implemented. For example a chest-worn monitor for cardiac and activity data may transmit data by radio transmission to a fixed receiver, for example next to an exercise machine for monitoring the user's heart rate and activity while exercising. Alternatively a chest-worn cardiac monitor (not incorporating an actigraph) could transmit cardiac data to a wrist-worn actigraph or to a fixed receiver.

Normal commercial electrode pad adhesives are designed to retain normal ECG leads and clips. In many monitoring applications, longer lead wires are required, and are separately supported to prevent pulling on the electrode pad. Preferably, the monitor embodying the invention is of similar weight to a single short ECG lead wire and clip. This means that the weight of the monitor does not pull on a normal electrode pad beyond the pad's design load. By being light weight, the monitor places a low load on the adhesive pad. Advantageously, this means that the monitor does not require the separate safety straps or wires that are deemed necessary for other, heavier, units mounted on multiple pads.

Preferably, the weight of the monitor is less than 50g, or particularly preferably less than 25g or 15g. In a preferred embodiment the monitor weighs about 10g or less.

Devices that mount on to multiple adhesive pads, or any device mounted on a single adhesive pad larger than a conventional ECG electrode pad, will disadvantageously require complicated mechanical arrangements, for example articulation or a flexible housing, to allow for the free movement of the body and resulting skin expansion and contraction. Advantageously, mounting at a single point, particularly on a standard ECG electrode pad, reduces the problems associated with free body movement and skin expansion and contraction. It is notable that this problem is taken into account in designing conventional ECG electrodes; these are of limited size in order to avoid problems arising from skin stretching and contraction.

These features of preferred embodiments of the invention may solve a number of problems in prior art heart-rate monitors. In prior art systems, for example, the transmission of data from a chest band using a radio link is subject to a wide range of interference, such as from electric motors, televisions, telephones etc., which typically leads to a large number of data points being lost and classed as "dropouts". Typically 10-20% of data points are lost in this way per day of monitoring. On-board storage of data within the apparatus embodying the invention solves this problem, as well as advantageously eliminating any electromagnetic transmissions from the apparatus which may interfere with other apparatus, such as medical apparatus.

In prior art systems, there is a lack of data storage facilities to allow for long-term accumulation of data, for example over periods of more than 24 hours. The memory in the monitor embodying the invention solves this problem.

The use of a chest-worn band for supporting a heart-rate monitor is not suitable for various categories of people, such as the very young, the very old and the obese, and is not comfortable for long-term use. The use of ECG electrodes to support and connect the monitor of the embodiment solves this problem and makes the monitor more comfortable to use.

Prior art heart-beat sensors are typically only used to measure heart-rate itself and not other important cardiac parameters such as inter-beat interval. The on-board processor of the embodiment can be programmed to measure any such parameters, particularly when combined with the use of ECG electrodes as these provide a very clear heart-beat signal.

In a preferred embodiment, when the monitor is supported on a user's chest or torso, the accelerometer should be oriented to detect vertical movements of the user's chest or torso. The inventors have found that this provides the most effective sensing of user movement, or activity.

The inventors have also found that the processing of the heart-beat sensor output to extract heart-rate and other cardiac information may advantageously be modified in response to the output from the accelerometer. Thus, for example, the gain and thresholds for ECG measurement are preferably adjusted based on the current user activity level measured by the accelerometer. During periods of activity, noise artefacts tend to be induced in the ECG signal by variations in skin potentials and using the activity data to improve the signal-to-noise ratio of the ECG signal helps to ensure a clean and uninterrupted data stream.

Although reference has been made to storing movement and heart-rate measurements in a memory housed within the apparatus of a preferred embodiment, other possibilities are envisaged within the scope of the invention. Thus, heart-beat or heart-rate data and movement data may be downloaded or transmitted to a remote display unit or data storage unit during use so that these signals may be monitored by a user, for example during exercise. If a user is engaged in a repetitive physical exercise such as, for example, running, output from the movement sensor may not only be valuable in combination with heart-rate measurement as described above but may also be used to determine the user's stride rate or the number of strides performed, for example.

An apparatus or method embodying the invention may be used for monitoring human or animal users.

Specific Embodiments and Best Mode of the Invention

Specific embodiments of the invention will now be described by way of example, with reference to the accompanying drawings, in which:
Figure 1 shows front, side and rear views of a first embodiment of the invention;
Figure 2 is a schematic diagram of the embodiment of Figure 1 coupled to two ECG electrodes for use;
Figure 3 is a schematic diagram of the embodiment of Figure 1 coupled to two rectangular ECG electrodes for use;
Figure 4 illustrates an embodiment of the invention comprising a monitor and a lead, in which the lead functions as an RF aerial;
Figure 5 is a block diagram of the circuitry of a monitor embodying the invention;
Figure 6 is a more detailed circuit diagram of the circuitry of Figure 5;
Figure 7 is a block diagram of an interface for coupling the monitor of Figure 5 to a PC;
Figure 8 is a flow diagram illustrating the operation of the monitor of Figure 5;
Figure 9 is a state diagram providing an overview of the operation of the monitor of Figure 5;
Figure 10 is a flow diagram illustrating the functionality of the monitor of Figure 5.
Figure 11 is a transverse section of an ECG electrode incorporating an electrode gel;
Figure 12 illustrates a zero-insertion-force mounting for a monitor embodying the invention; and
Figure 13 is a side view of a monitor embodying the invention mounted on an ECG pad.

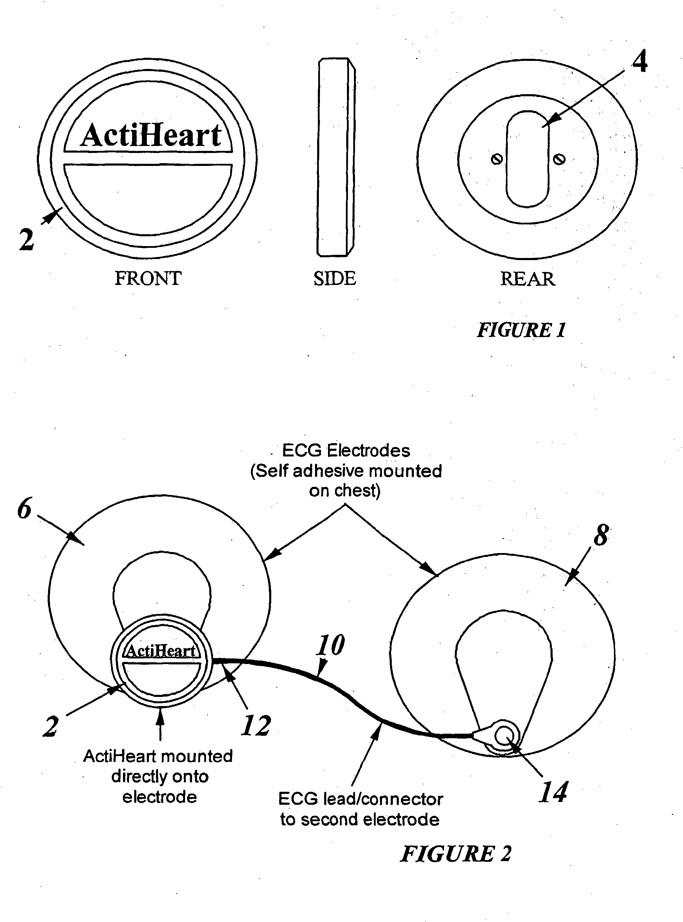

Figure 1 shows the external appearance of a housing of a monitor 2 according to a first embodiment of the invention, viewed from the front, side and rear. The monitor is substantially disc shaped, having a diameter of about 31 mm and a thickness of about 5.5 mm. The rear of the monitor comprises a recessed clip 4 which is removably attachable to an electrical contact of a conventional ECG electrode.

Figure 2 illustrates the monitor of Figure 1 in use. Two conventional ECG electrodes 6, 8 each comprise a circular adhesive pad which can be stuck to a user's chest. Each also comprises an electrical contact positioned near a lower edge of the pad and extending forwards from the pad.

The clip 4 of the monitor 2 mounts on the electrical contact of one electrode 6. The monitor comprises an electrical lead 10 for coupling to the other ECG electrode 8. The lead carries at one end a plug 12 which is removably insertable into a socket in one side of the monitor housing, and at the other end a clip 14 which is removably connectable to the ECG electrode contact.

For user comfort, the lead 10 should be longer than the distance between the ECG electrodes, to accommodate user movement.

The monitor comprises an accelerometer, as described below, which is primarily sensitive to movement in a particular direction. In the embodiment the accelerometer is mounted within the monitor so as to detect vertical motion of the user's chest, which requires that the monitor is mounted and retained in the correct orientation on the ECG pad. The correct orientation for mounting the monitor is indicated to the user by a marking on the monitor casing. Once fitted to the ECG electrode, the clip 4 holds the monitor in position. The lead connecting the monitor to the second ECG pad also helps to orient the monitor correctly.

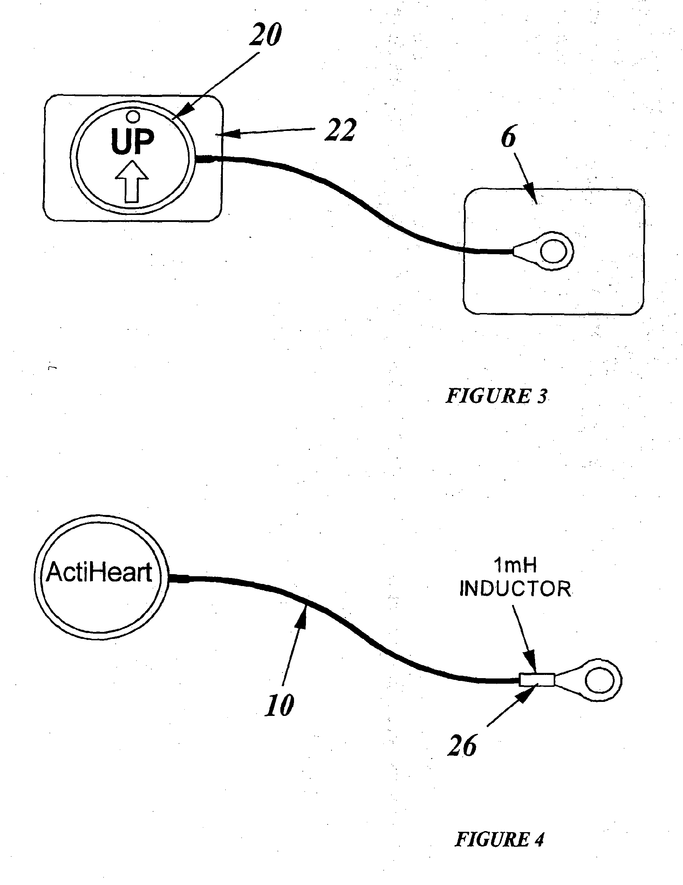

Figure 3 illustrates a similar monitor 20 mounted on conventional ECG electrodes 22, 24 of a different type, which are of generally rectangular shape.

Figure 4 illustrates a further embodiment in which the lead 10 coupling the monitor to a second ECG electrode acts as an RF (radio frequency) aerial, allowing the monitor to transmit such data to an RF receiver. In this embodiment the lead terminates at a 1mH inductor 26 to enhance its functionality as a aerial.

Figure 12 illustrates the clip 4, for securing the monitor of Figure 1 to an ECG electrode, in more detail. Similar clips may be used in the embodiments of Figures 2 to 4. This zero-insertion-force clip comprises a slider 70 in which an opening 71 is formed, the opening having an enlarged portion 72 at one end through which a conventional 4mm stud 74 of an ECG electrode may be received. An anvil 76 extends into the opening and must be manually withdrawn from the opening (or the slider moved relative to the anvil) against a spring force to allow the stud to enter the opening. From the enlarged portion 72 the opening tapers inwardly between two straight sides 78 at an acute angle to each other. After entry of the stud into the opening, the anvil (or the slider) is released and the anvil abuts the stud so that the spring force urges the stud between the tapering sides 78 of the opening. The spring force is designed to be high enough and the angle between the tapering sides small enough to provide a firm grip between the clip and the stud, to produce good electrical contact and to resist rotation of the monitor relative to the ECG electrode.

As shown in Figure 12 the slider is a plate approximately 1mm thick. This allows it to grip a lower neck portion 80 of the conventional ECG electrode stud (see Figure 11) and hold the base of the monitor flush with or close to the upper surface of the ECG electrode. This clip may advantageously allow zero insertion force and a much more secure mounting than the spring clips conventionally used to secure leads to ECG electrodes.

The monitors illustrated in Figures 1, 2, 3 and 4 are of diameter 31mm and thickness 5.5mm. However, a monitor embodying the invention should advantageously be less than 70mm, and particularly preferably less than 50mm in lateral dimension.

Preferably, the monitor is less than 35 mm in diameter (or lateral or vertical dimension). The preferred position for both actigraphy measurement and ECG electrode pad positioning is on or near the sternum. In order to fit comfortably between female breasts, the size of the monitor housing should be similar to or smaller than that of many normal ECG electrodes. Advantageously, a diameter of about 30mm, or a lateral dimension of about 30mm, is of this order.

In addition, the monitor should advantageously be less than 15mm in thickness and particularly preferably less than 10mm or less than 6mm in thickness. These dimensions aim to ensure user comfort.

Advantageously, these thickness dimensions mean that the monitor housing is of low profile. A low profile may, advantageously, render the monitor almost invisible when worn under clothing. The low profile may also reduce interactions between the monitor and clothing which would otherwise cause movement or tipping of the monitor, or even rip the monitor from its mounting. Interaction with clothing may cause undesirable movement of a monitor of larger diameter or a monitor having a greater thickness. Devices that hang from an ECG electrode by a wire may also suffer from the same excessive movement problem.

When the monitor is clipped to an electrode pad, there may be a gap between the monitor and the pad. Preferably, this gap is narrow. A large gap may allow the monitor to tip or allow clothing to rip the monitor from the pad. Tipping of the monitor is also undesirable as it may generate spurious movement data or affect the ability of the accelerometer to detect vertical movement. A narrow gap, or no gap, between monitor and pad may, advantageously, lessen these undesirable effects.

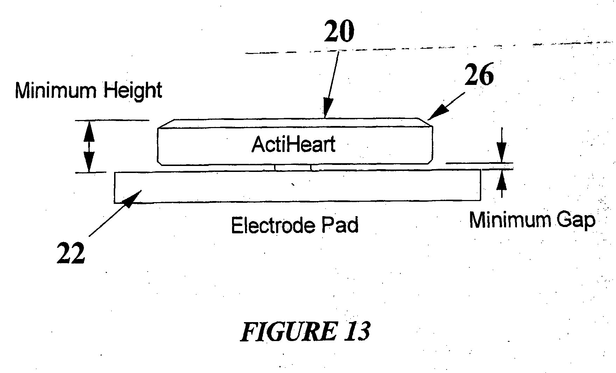

Figure 13 is a side view of a monitor 20 mounted on an ECG pad 22 as in Figure 3. The monitor is about 5mm thick and the support, or clip, fastening the monitor to the pad is positioned within the monitor housing so that the base of the monitor housing is flush with, or spaced by less than 1mm or 2mm from, the upper surface of the ECG electrode to minimise tipping of the monitor relative to the electrode. The outer rim 26 of the monitor housing is bevelled to reduce further the risk of the monitor catching on clothing.

Movements of the monitor, such as twisting or tipping, on the electrode pad may cause ECG noise artifacts. A small size and low profile may help to reduce excessive noise artifacts caused by movement of the monitor. The monitor may then, advantageously, be used during vigorous exercise without generating excessive noise artefacts.

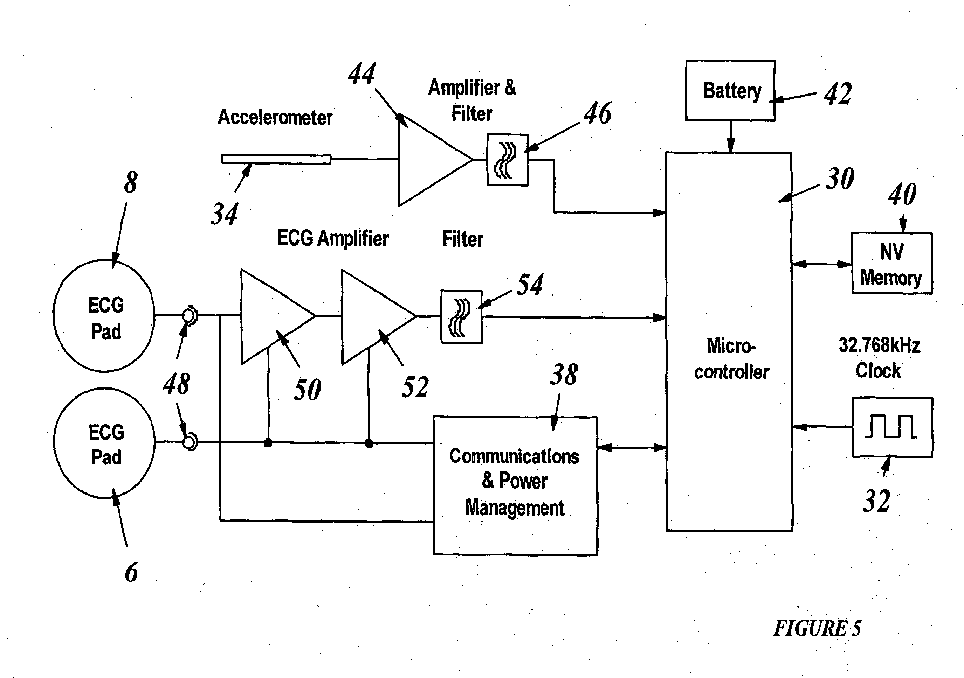

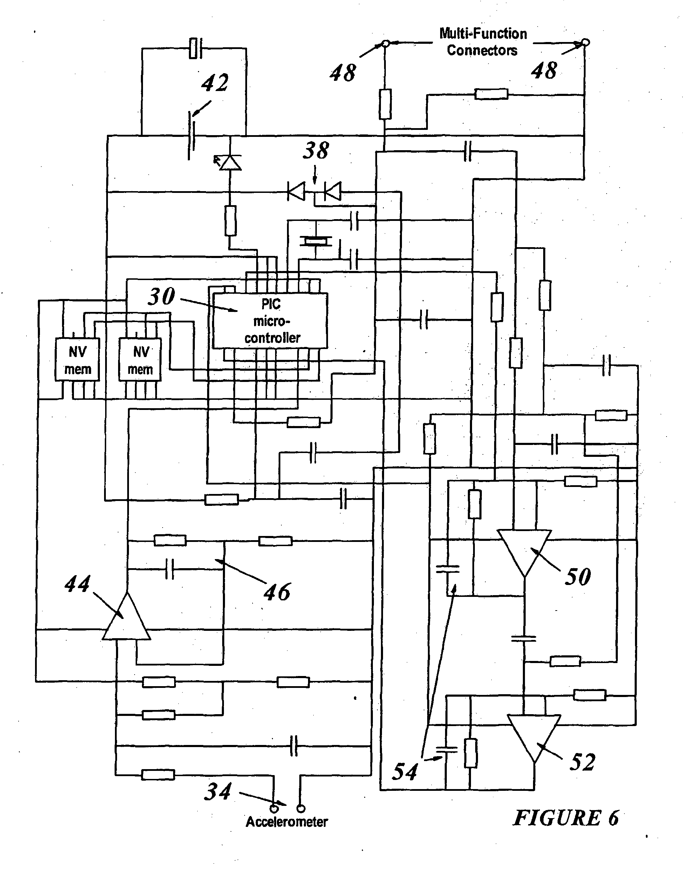

Figure 5 is a block diagram of a monitor circuit embodying the invention. Figure 6 is a more detailed circuit diagram corresponding to the block diagram of Figure 5.

The circuit comprises a microcontroller 30 which receives inputs from a clock (crystal oscillator) 32, an accelerometer 34, two ECG electrodes 6, 8 and a communications and power management module 38. The microcontroller 30 is also coupled to a memory 40 and a battery (re-chargeable coin cell) 42. All of these components are mounted on a printed circuit board (PCB) which is housed within a monitor casing or housing such as illustrated in Figures 1 to 4.

The accelerometer is a piezoelectric accelerometer, which is mounted on the PCB in a predetermined orientation such that it is most sensitive to motion in a predetermined direction when the PCB is housed in the monitor casing and the monitor is in use. For example, in the chest-mounted embodiments of figures 1 to 4, the accelerometer is oriented to be most sensitive to movement in the vertical plane (i.e. sensitive to physical movement in the up/down direction), during use when the user is upright. In this way, a good approximation of the physical activity of the user may be deduced. In other applications for sensing other movements of a human or animal body it may be desirable to mount the accelerometer in different predetermined orientations within the monitor casing.

The signal from the accelerometer is amplified by an amplifier 44 and filtered by a filter 46 before being input to an analog input of the microcontroller.

The ECG electrodes are usually attached to the mid-left region of the user's chest and the monitor is coupled between them. The monitor may comprise a small light emitting diode (LED) which flashes for several beats to indicate when an ECG signal is initially detected. It may additionally flash whenever a heartbeat is detected, allowing the user to confirm that the monitor is picking up an ECG signal.

Preferably, the LED flash is modulated at high frequency so that a light receiver can easily detect its light. Advantageously, this enables remote electrically-isolated short-range readout of the heart rate in fitness tests such as treadmill tests.

The signals from the ECG electrodes pass through two monitor contacts 48 and are amplified in two stages by two amplifiers 50, 52 and filtered by a filter 54 before being input to an analog input of the microcontroller.

The ECG signal is processed within the microcontroller to remove noise artefacts. As the monitor is totally self-contained, there are no problems with interference from radio frequency devices or other sources of electromagnetic interference.

The microcontroller uses a 4.0 MHz internal clock for instruction timing but uses an external 32.768 kHz oscillator, shown in Figure 5 as the clock 32, for real-time clock functions.

The communications and power management block 38 is coupled to the monitor contacts 48 and comprises discrete circuitry which allows various signal levels and frequencies at the contacts to be discriminated by the microcontroller. This allows the monitor contacts to be used as monitor inputs or outputs for multiple functions depending on the device to which the contacts are coupled. Thus, if the contacts are coupled to ECG electrodes, ECG signals can be identified and received by the microcontroller. If the contacts are coupled to an interface unit or reader as described below, the same contacts can be used by the microcontroller to download data, re-charge the battery, or other applications as described below.

The battery 42 is a surface-mounted manganese lithium secondary (re-chargeable) coin cell that provides up to 22 days of continuous operation from a full charge. During operation, the monitor may continuously record heart rate and physical activity at one minute intervals. All of the other components are also surface-mounted on the PCB to provide compact size, simplified production and increased reliability.

The circuit is provided with protection from reverse polarity connection, over-voltage and ESD (electro-static discharge). The ultra-low power and integrated nature of the monitor ensures no EMI (electro-magnetic) emissions.

The device is waterproof and can hence be worn continually to provide an uninterrupted data stream.

### Firmware

In the monitor, certain firmware (embedded software) is programmed into an internal ROM (read only memory) area of the microcontroller 30 and controls many of the monitor's functions. In particular, the firmware enables the sampling of signals from the accelerometer and the ECG electrodes under timed interrupts, with movement being sampled at 16 Hz and ECG at 128 Hz or 256 Hz. These signals are sampled at different rates to reflect the different rates at which the signals typically vary. The movement data are integrated over one minute epochs and stored into non-volatile memory 40. The heart rate data are stored as beats per minute in the non-volatile memory.

The microcontroller performs several signal processing functions and executes internal algorithms on the ECG data. The key processing functions are as follows. Dynamic threshold: the threshold for detection of the ECG R-wave pulse is dynamically adjusted within a window period to aid discrimination of true pulse signals during periods of high noise.

Variable gain: the gain and dV/dt (rate of change of voltage) thresholds for ECG measurement are adjusted based on the current user movement level detected by the accelerometer. During periods of movement, noise artefacts are induced by variations in the user's skin potentials. Using the movement data to improve the signal-to-noise ratio of the ECG signal helps to ensure a clean and uninterrupted data stream.

The monitor uses a digitally computed reference level to determine whether the dV/dt (rate of change of voltage) of the input signal meets the requirements of an ECG R-wave. This threshold varies with time and has an absolute minimum level to prevent spurius noise being seen as an R-wave. During periods of high activity the minimum threshold level is raised, as described above, to prevent spurius triggering on movement noise artifacts.

IBI Tracking: the inter-beat interval (IBI) is computed and used to update an internally stored histogram. The histogram contains discrete time windows and an IBI value falling within a histogram window causes the histogram to be incremented. An indication of variation of the inter-beat interval is very useful in determining certain medical conditions.

IBI variability logging: normal regular heart-rate data are stored as beats per minute. If serious variability is detected, the heart rate is automatically stored at a higher resolution to allow a more detailed analysis.

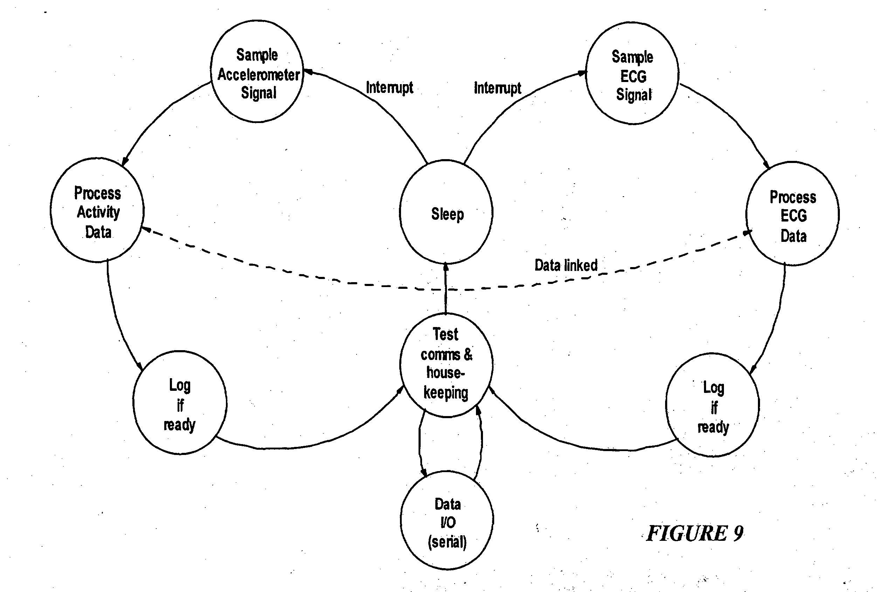

Figure 9 provides an overview of the firmware operation.

### Reader

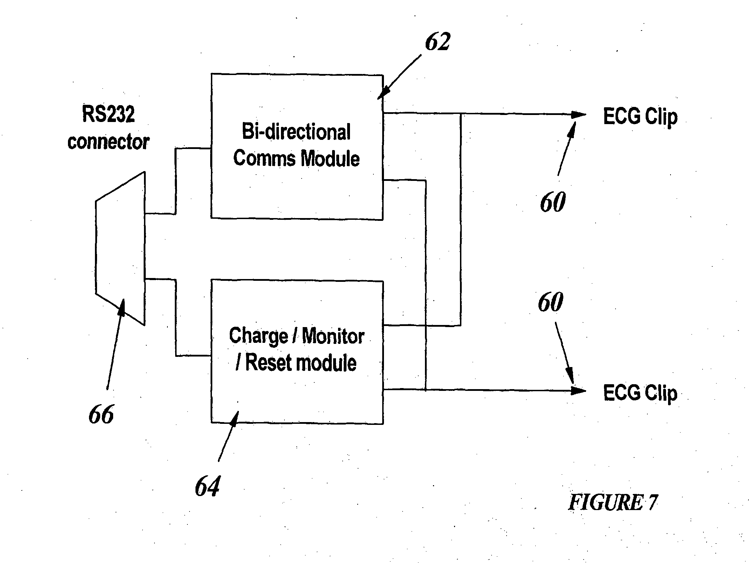

Figure 7 is a block diagram of a reader, or interface, for coupling the monitor to a PC. The reader comprises terminals 60 connectable to the ECG electrode contacts 48 of the monitor. Within the reader, these are connected to a bi-directional communications module 62 and a charge/monitor/re-set module 64. Each of these modules is connected by an RS232 connector 66, or other connector suitable for interfacing to a PC, such as a USB connector.

The reader is thus a small module that contains the electronics necessary for level shifting to and from RS232 (or USB) in addition to providing control signals for power management of the monitor. Once the monitor is connected to the reader via the ECG leads, after simply unclipping the monitor from the ECG electrodes and connecting the same contacts to the reader, bi-directional communications may take place between the monitor and the serial port of the PC. As well as allowing data to be downloaded from the monitor to the PC, the reader can also charge the monitor battery, drawing power from the PC serial port or optionally from a plug-in mains adaptor.

### Software

This software runs on a PC having a serial port to which the monitor may be coupled via the reader described above. The software is a 32-bit Windows application written in Visual Basic with an underlying database used for data management. The software has the following broad functions.

Store details of users and test data in structured and manageable database tables.

Write user and test parameters to the monitor.

Read logged data from the monitor.

Present reports in a user-friendly and flexible manner.

Provide portable data storage. This means that data can be exported to other software packages for additional analysis.

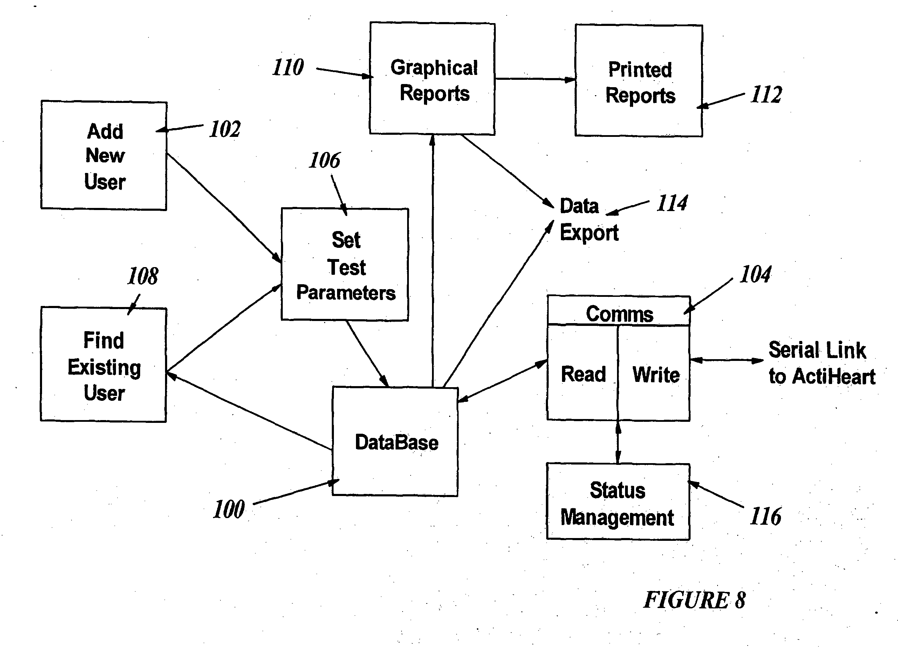

Figure 8 shows a block diagram of the software structure.

A core database 100, which is Access compatible, contains tables for user information such as name, date of birth, height etc. The database also has tables to contain downloaded heart rate, or other cardiac data, and movement, or activity, data. The tables have relational interlinking and the software generates queries to present users seamlessly with the correct downloaded data.

When a new user is added 102, their personal details are stored into the database. A set of test-specific parameters (i.e. user weight, test start date and time etc.) are also set and stored 106. Alternatively, existing users may be located 108 from the database using search facilities and the test parameters then set or selected. Set-up information is then transferred to the monitor by means of a communications module 104 and a serial link (coupled through the reader to the monitor).

The communications module 104 also controls monitor status management 116, including monitoring the level of charge in the monitor battery and re-setting the monitor microcontroller where required.

In addition, data may be downloaded through the serial link under the control of the communications module from the monitor to the database 100 and viewed using a graphical reports module 110. Graphical reports may be printed 112 or data may be exported 114 directly from the database or via the clipboard from the graphical reports module.

### Functionality of the Monitor Contacts

As described above, the monitor comprises two electrical contacts, which can be coupled either to ECG electrodes for heart-beat sensing or to an external device such as the reader for various other purposes.

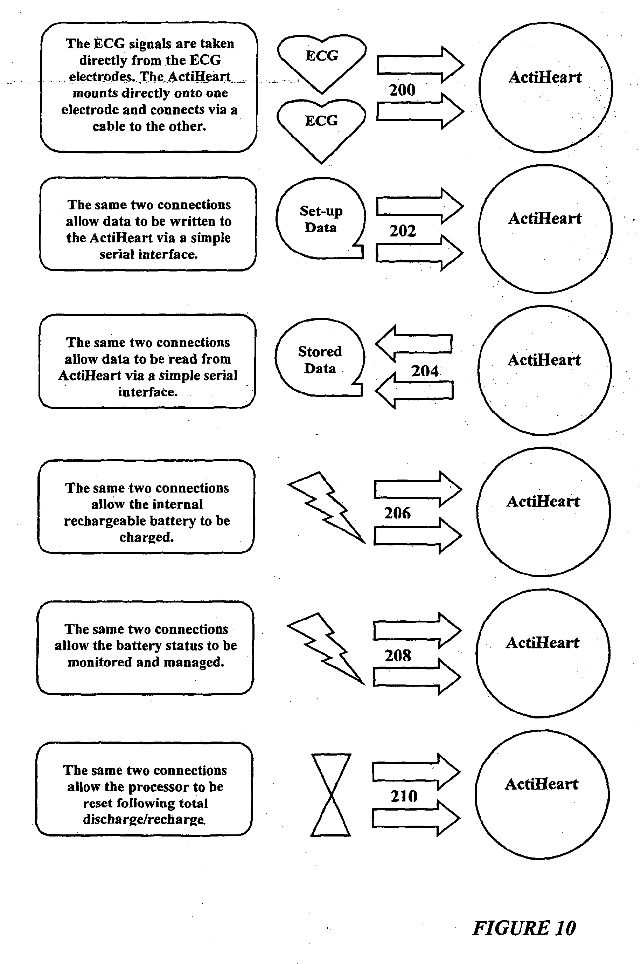

Figure 10 illustrates the various functions of the monitor contacts.

In total, the two contacts for the ECG electrodes are also used for five other functions: reading data, writing data, charging battery, power management and re-setting the CPU of the microcontroller. This shared functionality of connections allows greatly reduced size and complexity of the electronics of the monitor and provides a simplified user interface.

As shown in Figure 10, when the monitor contacts are coupled to ECG electrodes (200), the ECG signals are taken directly from those electrodes. In a preferred embodiment, the monitor mounts directly onto one electrode and connects via a cable to the other.

When the monitor contacts are coupled to a PC via a serial interface (202), data can be written by the PC to the monitor. This allows set-up data to be written to the monitor, including user identification and any other desired test parameters.

Similarly, when the monitor contacts are coupled to the serial interface (204), data can be read from the monitor by the PC. This allows stored movement (activity) and cardiac data to be downloaded.

When the monitor contacts are coupled either to a suitable serial interface or to a battery charger (206), the same connections allow the internal re-chargeable battery to be charged.

When the monitor contacts are coupled to a suitable interface, such as the reader described above, battery status can be monitored and managed (208).

Finally, when the monitor contacts are coupled to a suitable interface such as the reader described above, the micro-controller can be re-set (210) following a total discharge or re-charge of the battery.

## Claims

1. A monitor (2) for monitoring a user's heart, comprising;
a support means (4) for securing the monitor in position for sensing the user's heart, beat, the support means being for attachment to a single adhesive ECG electrode (6) both to support the monitor and for receiving electrical signals from the ECG electrode;
a means (10) for electrically coupling the monitor to a second ECG electrode (8) for receiving signals therefrom;
a cardiac sensor (50,52,54) for receiving signals from the ECG electrodes,
a processor (30) coupled to the cardiac sensor for generating cardiac data, and
contacts (48) for making electrical contact with the ECG electrodes (6,8), **characterised in that**
the monitor (2) is configured to transfer data from the monitor,
and/or reset or reprogram the monitor, and/or recharge a battery (42) for powering the monitor via the same contacts (48).

2. A monitor according to claim 1, in which the coupling means (10) comprises an electrical lead extending from a housing of the monitor (2) or a socket in the housing of the monitor (2) for receiving an electrical lead.

3. A monitor according to any preceding claim, in which the maximum lateral dimension of the monitor is 35 mm or less, or is preferably less than or equal to the maximum lateral dimension of the ECG electrode (6), and/or in which the monitor (2), in use, does not extend beyond an outer edge of the ECG electrode (6), and/or in which the weight of the monitor (2) is less than 50 grams.

4. A monitor according to any preceding claim, comprising a memory (40) coupled to an output of the processor (30) for storing the cardiac data;
and/or in which the processor (30) is configured to generate inter-beat interval data from signals it receives from the cardiac sensor (50,52,54).

5. A monitor according to any preceding claim, further comprising an accelerometer (34) coupled to the processor (30), the processor being configured to generate movement data, in which the monitor (2) in use is preferably secured to the chest or torso of the user so that the accelerometer (34) is oriented to sense vertical movements of the user's chest or torso;
and in which the processor (30) optionally is configured to processe signals it receives from the cardiac sensor (50,52,54) according to a predetermined parameter in order to generate the cardiac data and is configured to modify that parameter in response to signals it receives from the accelerometer (34);
and/or in which a parameter, such as a gain parameter or a threshold voltage, used in deriving the cardiac data from an output of the cardiac sensor is variable in response to an output from the accelerometer or a movement or activity parameter derived therefrom.

6. A monitor according to any preceding claim, which is of small size and weight so as to be comfortable for a user to wear for extended data sampling periods.

7. A monitor system comprising a monitor (2) in accordance with any one of the preceding claims and a monitor reader,
wherein the reader comprises:
terminals (60) connectable to the contact (48) to the monitor,
a connector (66), suitable for interfacing to a PC,
a bi-directional communications module (62) connected between the terminals and the connector, and
a charge/monitor/re-set module (64) connected between the terminals and the connector.

8. method of providing data signals from or power signals to a monitor, the monitor (2) comprising:
a support means (4) for securing the monitor in position for sensing the user's heart beat, the support means being for attachment to a single adhesive ECG electrode (6) both to support the monitor (2) and for receiving electrical signals from the ECG electrode;
a means (10) for electrically coupling the monitor to a second ECG electrode (8) for receiving signals therefrom;
a cardiac sensor (50,52,54) for receiving signals from the ECG electrodes; and
a processor (30) coupled to the cardiac sensor for generating cardiac data,
wherein the monitor further includes contacts (48) for making electrical contact with the two ECG electrodes (6,8),
comprising the step of:
using the contacts (48) to couple the monitor (2) to an external device for transferring data from the monitor (2), and/or resetting or reprogramming the monitor, and/or recharging a battery (42) for powering the monitor.

## Patentansprüche

1. Monitor (2) zur Überwachung des Herzes eines Benutzers, umfassend:
ein Tragemittel (4) zum Befestigen des Monitors in Position zur Erfassung des Herzschlags des Benutzers, wobei das Tragemittel zur Anbringung an einer einzelnen EKG-Klebeelektrode (6) sowohl zum Tragen des Monitors als auch zum Empfangen elektrischer Signale von der EKG-Elektrode ist,
ein Mittel (10) zum elektrischen Koppeln des Monitors mit einer zweiten EKG-Elektrode (8) zum Empfangen von Signalen von ihr,
einen Herzsensor (50, 52, 54) zum Empfangen von Signalen von den EKG-Elektroden,
einen Prozessor (30), der mit dem Herzsensor gekoppelt ist, zum Erzeugen von Herzdaten und
Kontakte (48) zum Herstellen eines elektrischen Kontakts mit den EKG-Elektroden (6, 8), **dadurch gekennzeichnet, dass**
der Monitor (2) konfiguriert ist zum Übertragen von Daten von dem Monitor und/oder zum Rücksetzen oder zum Umprogrammieren des Monitors und/oder zum Aufladen einer Batterie (42) zum Versorgen des Monitors über dieselben Kontakte (48) mit Energie.

2. Monitor nach Anspruch 1, bei dem das Kopplungsmittel (10) ein elektrisches Kabel umfasst, das von einem Gehäuse des Monitors (2) oder einer Buchse in dem Gehäuse des Monitors (2) zum Aufnehmen eines elektrischen Kabels verläuft.

3. Monitor nach einem der vorhergehenden Ansprüche, bei dem die maximale seitliche Dimension des Monitors 35 mm oder weniger ist oder vorzugsweise so groß wie die maximale seitliche Dimension der EKG-Elektrode (6) oder kleiner ist und/oder bei dem sich der Monitor (2) im Gebrauch nicht über einen Außenrand der EKG-Elektrode (6) hinaus erstreckt und/oder bei dem das Gewicht des Monitors (2) kleiner als 50 Gramm ist.

4. Monitor nach einem der vorhergehenden Ansprüche, der einen mit einem Ausgang des Prozessors (30) gekoppelten Speicher (40) zum Speichern der Herzdaten aufweist
und/oder bei dem der Prozessor (30) konfiguriert ist zum Erzeugen der R-R-Intervalldaten anhand von Signalen, die er von dem Herzsensor (50, 52, 54) empfängt.

5. Monitor nach einem der vorhergehenden Ansprüche, der ferner einen Beschleunigungsmesser (34) aufweist, der mit dem Prozessor (30) gekoppelt ist, wobei der Prozessor zum Erzeugen von Bewegungsdaten konfiguriert ist, bei dem der Monitor (2) im Gebrauch vorzugsweise an der Brust oder dem Rumpf des Benutzers befestigt ist, damit der Beschleunigungsmesser (34) zum Erfassen vertikaler Bewegungen der Brust oder des Rumpfes des Benutzers ausgerichtet ist,
und bei dem der Prozessor (30) wahlweise konfiguriert ist zum Verarbeiten von Signalen, die er von dem Herzsensor (50, 52, 53) empfängt, gemäß einem vorbestimmten Parameter, um die Herzdaten zu erzeugen, und konfiguriert ist zum Modifizieren dieses Parameters als Reaktion auf Signale, die er von dem Beschleunigungsmesser (34)empfängt,
und/oder bei dem ein Parameter wie ein Verstärkungsparameter oder eine Schwellenspannung, die beim Ableiten der Herzdaten von einem Ausgang des Herzsensors verwendet wird, als Reaktion auf einen Ausgang von dem Beschleunigungsmesser oder einen davon abgeleiteten Bewegungs- oder Aktivitätsparameter variabel ist.

6. Monitor nach einem der vorhergehenden Ansprüche, dessen Größe und Gewicht klein ist, damit er für einen Benutzer zum Tragen für ausgedehnte Datenabtastzeiten komfortabel ist.

7. Monitorsystem, umfassend einen Monitor (2) nach einem der vorhergehenden Ansprüche und ein Monitorlesegerät,
wobei das Lesegerät Folgendes umfasst:
Anschlüsse (60), die mit den Kontakten (48) des Monitors verbindbar sind,
einen Verbinder (66), der zum Anschluss an einen PC geeignet ist,
ein bidirektionales Kommunikationsmodul (62), das zwischen die Anschlüsse und den Verbinder geschaltet ist, und
ein Lade-/Monitor-/Reset-Modul (64), das zwischen die Anschlüsse und den Verbinder geschaltet ist.

8. Verfahren zum Bereitstellen von Datensignalen von oder Energieversorungssignalen zu einem Monitor,
wobei der Monitor (2) Folgendes umfasst:
ein Tragemittel (4) zum Befestigen des Monitors in Position zur Erfassung des Herzschlags des Benutzers, wobei das Tragemittel zur Anbringung an einer einzelnen EKG-Klebeelektrode (6) sowohl zum Tragen des Monitors als auch zum Empfangen elektrischer Signale von der EKG-Elektrode ist,
ein Mittel (10) zum elektrischen Koppeln des Monitors mit einer zweiten EKG-Elektrode (8) zum Empfangen von Signalen von ihr,
einen Herzsensor (50, 52, 54) zum Empfangen von Signalen von den EKG-Elektroden,
einen Prozessor (30), der mit dem Herzsensor gekoppelt ist, zum Erzeugen von Herzdaten,
wobei der Monitor ferner Kontakte (48) zum Herstellen eines elektrischen Kontakts mit den zwei EKG-Elektroden (6, 8) beinhaltet,
umfassend den Schritt des:
Nutzens der Kontakte (48) zum Koppeln des Monitors (2) mit einer externen Vorrichtung zum Übertragen von Daten von dem Monitor (2) und/oder zum Rücksetzen oder Umprogrammieren des Monitors und/oder zum Aufladen einer Batterie (42) zum Versorgen des Monitors mit Energie.

## Revendications

1. Moniteur (2) pour surveiller le coeur d'un utilisateur, comprenant :
un moyen de support (4) pour fixer en place le moniteur pour détecter le battement de coeur de l'utilisateur, le moyen de support étant destiné à être fixé à une électrode d'ECG unique adhésive (6) à la fois pour supporter le moniteur et pour recevoir des signaux électriques de l'électrode d'ECG ;
un moyen (10) pour coupler électriquement le moniteur à une seconde électrode d'ECG (8) pour recevoir des signaux de celle-ci ;
un capteur cardiaque (50, 52, 54) pour recevoir des signaux des électrodes d'ECG ;
un processeur (30) couplé au capteur cardiaque pour générer des données cardiaques, et
des contacts (48) pour établir un contact électrique avec les électrodes d'ECG (6, 8), **caractérisé en ce que** le moniteur (2) est configuré pour transférer les données provenant du moniteur et/ou réinitialiser ou reprogrammer le moniteur, et/ou recharger une batterie (42) pour alimenter électriquement le moniteur par l'intermédiaire des mêmes contacts (48).

2. Moniteur selon la revendication 1, dans lequel le moyen de couplage (10) comprend un câble électrique s'étendant depuis un boîtier du moniteur (2) ou une prise femelle dans le boîtier du moniteur (2) destinée à recevoir un câble électrique.

3. Moniteur selon l'une quelconque des revendications précédentes, dans lequel la dimension latérale maximum du moniteur est de 35 mm ou moins, ou est de préférence inférieure ou égale à la dimension latérale maximale de l'électrode d'ECG (6), et/ou le moniteur (2), durant l'utilisation, ne s'étendant pas au-delà d'un bord externe de l'électrode d'ECG (6) et/ou dans lequel le poids du moniteur (2) est inférieur à 50 grammes.

4. Moniteur selon l'une quelconque des revendications précédentes, comprenant une mémoire (40) couplée à une sortie du processeur (30) pour mémoriser les données cardiaques ;
et/ou dans lequel le processeur (30) est configuré pour générer des données d'intervalles entre battements à partir des signaux qu'il reçoit du capteur cardiaque (50, 52, 54).

5. Moniteur selon l'une quelconque des revendications précédentes, comprenant en outre un accéléromètre (34) couplé au processeur (30), le processeur étant configuré pour générer des données de mouvement, dans lequel le moniteur (2) durant l'utilisation est de préférence fixé sur la poitrine ou le torse de l'utilisateur de telle sorte que l'accéléromètre (34) soit orienté pour détecter les mouvements verticaux de la poitrine ou du torse de l'utilisateur ;
et dans lequel le processeur (30) est configuré optionnellement pour traiter les signaux qu'il reçoit du capteur cardiaque (50, 52, 54) en fonction d'un paramètre prédéterminé afin de générer les données cardiaques et est configuré pour modifier ce paramètre en réponse aux signaux qu'il reçoit de l'accéléromètre (34) ;
et/ou dans lequel un paramètre, tel qu'un paramètre de gain ou une tension de seuil, utilisé dans la dérivée des données cardiaques depuis une sortie du capteur cardiaque est variable en réponse à une sortie de l'accéléromètre ou d'un paramètre de mouvement ou d'activité dérivé de celle-ci.

6. Moniteur selon l'une quelconque des revendications précédentes, lequel est de petite taille et de faible poids de manière à ce que l'utilisateur puisse le porter confortablement pendant de longues périodes d'échantillonnage de données.

7. Système de contrôle comprenant un moniteur (2) selon l'une quelconque des revendications précédentes et un lecteur de moniteur,
dans lequel le lecteur comprend :
des bornes (60) pouvant être connectées aux contacts (48) du moniteur,
un connecteur (66), adapté pour l'interfaçage avec un ordinateur individuel,
un module de communications bidirectionnelles (62) connecté entre les bornes et le connecteur, et
un module de charge/contrôle/réinitialisation (64) connecté entre les bornes et le connecteur.

8. Procédé de fourniture de signaux de données depuis un moniteur ou de signaux de puissance à un moniteur,
le moniteur (2) comprenant :
un moyen de support (4) pour fixer en place le moniteur pour détecter le battement de coeur de l'utilisateur, le moyen de support étant destiné à être fixé à une électrode d'ECG unique adhésive (6) à la fois pour supporter le moniteur (2) et pour recevoir des signaux électriques de l'électrode d'ECG ;
un moyen (10) pour coupler électriquement le moniteur à une seconde électrode d'ECG (8) pour recevoir des signaux de celle-ci ;
un capteur cardiaque (50, 52, 54) pour recevoir des signaux des électrodes d'ECG ; et
un processeur (30) couplé au capteur cardiaque pour générer des données cardiaques,
le moniteur comportant en outre des contacts (48) pour établir un contact électrique avec les deux électrodes d'ECG (6, 8),
comprenant l'étape consistant à :
utiliser les contacts (48) pour coupler le moniteur (2) à un dispositif externe pour transférer les données provenant du moniteur (2), et/ou réinitialiser ou reprogrammer le moniteur, et/ou recharger une batterie (42) pour alimenter électriquement le moniteur.
